(19) Europäisches Patentamt / European Patent Office / Office européen des brevets

(11) **EP 3 728 583 B1**

(12) **EUROPEAN PATENT SPECIFICATION**

(45) Date of publication and mention
of the grant of the patent:
**01.03.2023 Bulletin 2023/09**

(21) Application number: **18814629.4**

(22) Date of filing: **12.12.2018**

(51) International Patent Classification (IPC):
*C12N 15/10* (2006.01)    *C12N 9/22* (2006.01)
*C12N 15/75* (2006.01)

(52) Cooperative Patent Classification (CPC):
**C12N 15/102; C12N 15/75;** C12Q 2521/301

(86) International application number:
**PCT/EP2018/084463**

(87) International publication number:
**WO 2019/121192 (27.06.2019 Gazette 2019/26)**

(54) **COUNTER-SELECTION BY INHIBITION OF CONDITIONALLY ESSENTIAL GENES**

GEGENAUSWAHL DURCH HEMMUNG BEDINGT ESSENTIELLER GENE

CONTRE-SÉLECTION PAR INHIBITION DE GÈNES ESSENTIELS DE MANIÈRE CONDITIONNELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2017 EP 17210256**

(43) Date of publication of application:
**28.10.2020 Bulletin 2020/44**

(73) Proprietor: **Novozymes A/S**
**2880 Bagsvaerd (DK)**

(72) Inventors:
• **RASMUSSEN, Michael, Dolberg**
**2880 Bagsvaerd (DK)**
• **PEDERSEN, Poul, Erik**
**2880 Bagsvaerd (DK)**

(74) Representative: **NZ EPO Representatives**
**Krogshoejvej 36**
**2880 Bagsvaerd (DK)**

(56) References cited:
WO-A1-2015/065964    WO-A1-2015/188109
WO-A1-2017/118720    WO-A1-2017/136629
WO-A1-2017/191210    WO-A2-2016/011080
WO-A2-2016/130600    WO-A2-2016/205745

• **TING-WEI WILL CHIANG ET AL: "CRISPR-Cas9D10A nickase-based genotypic and phenotypic screening to enhance genome editing", SCIENTIFIC REPORTS, vol. 6, no. 1, 15 April 2016 (2016-04-15), XP055463911, DOI: 10.1038/srep24356**
• **LUN YAO ET AL: "Multiple Gene Repression in Cyanobacteria Using CRISPRi", ACS SYNTHETIC BIOLOGY, vol. 5, no. 3, 18 March 2016 (2016-03-18), pages 207-212, XP055405676, Washington, DC, USA ISSN: 2161-5063, DOI: 10.1021/acssynbio.5b00264**
• **LV LI ET AL: "Application of CRISPRi for prokaryotic metabolic engineering involving multiple genes, a case study: Controllable P(3HB-co-4HB) biosynthesis", METABOLIC ENGINEERING, ACADEMIC PRESS, US, vol. 29, 31 March 2015 (2015-03-31), pages 160-168, XP029590702, ISSN: 1096-7176, DOI: 10.1016/J.YMBEN.2015.03.013**
• **PETERS JASON M ET AL: "A Comprehensive, CRISPR-based Functional Analysis of Essential Genes in Bacteria", CELL, CELL PRESS, vol. 165, no. 6, 26 May 2016 (2016-05-26), pages 1493-1506, XP029567375, ISSN: 0092-8674, DOI: 10.1016/J.CELL.2016.05.003**
• **LUKE?A. GILBERT ET AL: "Genome-Scale CRISPR-Mediated Control of Gene Repression and Activation", CELL, vol. 159, no. 3, 1 October 2014 (2014-10-01), pages 647-661, XP055247644, ISSN: 0092-8674, DOI: 10.1016/j.cell.2014.09.029**

**(Cont. next page)**

Remarks:
The complete document including Reference Table(s) and the Sequence Listing(s) can be downloaded from the EPO website

## Description

### Reference to a Sequence Listing

This application contains a Sequence Listing in computer-readable form, which is incorporated herein by reference.

### Field of the Invention

The present invention relates to method for counter-selection by inhibition of conditionally essential genes.

### Background of the Invention

The so-called CRISPR-Cas9 genome editing system has been widely used as a tool to modify the genomes of a number of organisms. The power of the CRISPR-Cas9 system lies in its simplicity to target and edit down to a single base pair in a specific gene of interest. The Cas9 protein is a dual-RNA guided endonuclease, with the nuclease activity being directed by so-called guide-RNA (gRNA) molecules. The choice of Cas9 target sequence is made by changing a 20 bp sequence window of the gRNA to match the target DNA sequence. When complexed with the gRNA molecule, the Cas9 protein will then bind its target DNA sequence and create a double-stranded break using two catalytic domains. Cas9 may be further engineered to contain a single amino acid mutation in either one of the two catalytic domains. In this case, Cas9 functions as a nickase, i.e. with single-strand cleavage activity.

In addition to its use within genome editing, the CRISPR-Cas9 system has also been used for control of gene expression. This application, often referred to as CRISPR inhibition or CRISPRi, allow sequence-specific repression or activation of a gene. CRISPR interference utilizes a catalytically inactive (dead) Cas9 variant (termed Cas9d) lacking endonuclease activity. The Cas9d-gRNA complex retains the ability to bind to the target DNA sequence, but cannot introduce any breaks in the DNA strand. By varying the gRNA sequence, one can control the target DNA sequence for the Cas9d-gRNA complex and thereby regulate the expression of virtually any gene in any organism provided a functional PAM sequence is present.

Within industrial biotechnology, there is a continued need for robust and effective selection systems suitable for development of optimized production hosts. Given the versatility and precision of the CRISPR-Cas9 technology, it has been speculated that this system could be harnessed for counter-selection purposes. However, attempts of utilizing the CRISPR-Cas9 technology for direct selection has so far been difficult. This is especially true for bacterial host cells, since many prokaryotic organisms are very sensitive to the endonuclease activity of the Cas9-gRNA complex due to the inefficient repair mechanisms for double-stranded (DS) breaks by non-homologous end-joining (NHEJ) systems that are known from eukaryotes (see, *e.g.*, Su et al., Scientific Reports 2016, 6, 37895; Altenbuchner, Applied and Environmental Microbiology 2016, 82, pp. 5421-5427; Peters et al., Current Opinion in Microbiology 2015, 27, pp. 121-126; Aravind and Koonin, Genome Research 2001, 11, pp. 1365-1374).

Furthermore, the direct selection using the CRISPR-Cas9 technology will be increasingly difficult if more than one site is targeted for DS breaks.

Many researchers have reported successful integration of a gene of interest (GOI) by homologous recombination (HR) into a gRNA target on chromosome and then introduce CRISPR-Cas9 activity for DS breaks to kill the cells which has retained the original gRNA target sequence. In this way, it is possible to efficiently enrich for cells which has received the GOI. However, the timing of these events of HR and DS activity are very important. The CRISPR-Cas9 complex is very active in generating DS breaks and should not be expressed until homologous recombination has occurred and removed the gRNA target.

### Summary of the Invention

The present invention provides means and methods for utilizing the versatility and precision of the CRISPR-Cas9 technology in a selection system suitable for bacterial host cells.

Thus, in a first aspect, the present invention relates to a method for inserting at least one polynucleotide of interest into the genome of a host cell, the method comprising the steps of:

a) providing a host cell comprising in its genome:

i. a polynucleotide encoding a selectable marker comprising a target sequence flanked by a functional PAM sequence for a Class-II Cas9 protein;
ii. at least one polynucleotide encoding a gRNA that is at least 80% complementary to and capable of hybridizing to the target sequence; and

iii. a polynucleotide encoding a nuclease-null variant of a Class-II Cas9 protein capable of interaction with the gRNA and binding to the target sequence, whereby expression of the selectable marker is repressed;

b) transforming said host cell with at least one polynucleotide of interest and capable of inactivating the at least one polynucleotide encoding the gRNA;

c) selecting for the trait conferred by the selectable marker; and

d) identifying a transformed host cell, wherein the at least one polynucleotide encoding the gRNA has been inactivated by the at least one polynucleotide of interest.

In a second aspect, the present invention relates to a method for inserting at least two different polynucleotides of interest into the genome of a host cell, the method comprising the steps of:

a) providing a host cell comprising in its genome:

i. at least two polynucleotides encoding at least two different selectable markers, each comprising a different target sequence flanked by a functional PAM sequence for a Class-II Cas9 protein;

ii. at least two polynucleotides encoding at least two gRNAs that are at least 80% complementary to and capable of hybridizing to the at least two different target sequences;

iii. a polynucleotide encoding a nuclease-null variant of a Class-II Cas9 protein capable of interacting with the at least two gRNAs and binding to the at least two different target sequences, whereby expression of the two different selectable markers is repressed;

b) transforming said host cell with at least two different polynucleotides of interest, said polynucleotides being capable of inactivating the at least two polynucleotides encoding the at least two gRNAs; and

c) selecting for the traits conferred by the at least two different selectable markers; and

d) identifying a transformed host cell, wherein the at least two polynucleotides encoding the at least two gRNAs have been inactivated by the at least two different polynucleotides of interest.

## Brief Description of the Figures

Figure 1 shows the bglC-cas9d *locus* in the PP3811-cas9d strain.

Figure 2 shows the gnt-dsRED-gDNA(cat) *locus* in PP3811-gDNA1 strain.

Figure 3 shows the amyL-dsRED-gDNA(cat) *locus* in PP3811-gDNA2 strain.

Figure 4 shows the lacA2-dsRED-gDNA(cat) *locus* in the PP3811-gDNA3 strain.

Figure 5 shows the gnt *locus* after integration of amyL in PP3811-amyL3.

Figure 6 shows the amyL *locus* after re-integration of amyL in PP3811-amyL3.

Figure 7 shows the lacA2 *locus* after integration of amyL in PP3811-amyL3.

Figure 8 shows a schematic drawing of the PP3811-gDNA3 strain.

Figure 9 shows the pPPamyL-attP plasmid.

Figure 10 shows the pSJ14072 plasmid.

## Definitions

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Conditionally essential gene:** A conditionally essential gene or locus may function as a selectable marker. Examples of bacterial conditionally essential selectable markers are the *dal* genes from Bacillus subtilis or Bacillus licheniformis, that are only essential when the bacterium is cultivated in the presence of D-alanine; or the genes encoding enzymes involved in the removal of UDP-galactose from the bacterial cell when the cell is grown in the presence of galactose. Non-limiting examples of such genes are those from B. *subtilis* or B. *licheniformis* encoding UTP-dependent phosphorylase (EC 2.7.7.10), UDP-glucose-dependent uridylyltransferase (EC 2.7.7.12), or UDP-galactose epimerase (EC 5.1.3.2). If an essential gene or *locus* is inactivated, it will render the resulting strain with a deficiency, *e.g.* being unable to metabolize a specific carbon-source, or a growth requirement, *e.g.*, becoming amino acid auxotrophic, or becoming sensitive to a given stress. Non-limiting examples of conditionally essential genes are D-alanine racemase-encoding genes, xylose isomerase-encoding genes, genes of the gluconate operon. Preferably the conditionally essential gene are chosen from the group consisting of *dal, lysA, araA, galE, antK, metC, xylA, gntP, gntK, glpD, glpF, glpK, glpP, lacA2, hisC, gapA,* and *aspB.*

**Control sequences:** The term "control sequences" means nucleic acid sequences necessary for expression of a polynucleotide encoding a mature polypeptide of the present invention. Each control sequence may be native (i.e., from the same gene) or foreign (i.e., from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Host cell:** The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., recombinant production in a host cell; multiple copies of a gene encoding the substance; and use of a stronger promoter than the promoter naturally associated with the gene encoding the substance). An isolated substance may be present in a fermentation broth sample; e.g. a host cell may be genetically modified to express the polypeptide of the invention. The fermentation broth from that host cell will comprise the isolated polypeptide.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice *et al.,* 2000, *supra),* preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(Identical\ Deoxyribonucleotides\ x\ 100)/(Length\ of\ Alignment - Total\ Number\ of\ Gaps\ in\ Alignment)$$

**Detailed Description of the Invention**

The present invention provides means and methods for utilizing the versatility and precision of the CRISPR-Cas9 technology in a selection system suitable for bacterial host cells. By using the DNA sequence encoding the gRNA (denoted 'gDNA') in CRISPRi as an indirect counter-selectable marker, the present inventors have shown that multiple gene copies can be inserted into the genome of a host cell by selection for the absence of the gDNA encoding the gRNA.

As illustrated in the Examples herein, a suitable selection system may be based on an antibiotics resistance gene such as the *cat* gene that confers resistance to chloramphenicol. A host cell comprising a polynucleotide encoding the cat gene as well as a polynucleotide encoding a nuclease-null variant of a Class-II Cas9 protein and a polynucleotide encoding a gRNA directed towards the *cat* gene will thus only grow only in the absence of chloramphenicol, since the Cas9d-gRNA complex will repress expression of the *cat* gene. As long as the nuclease-null variant of a Class-II Cas9 protein and the gRNA is expressed by the host cell, the host cell remain sensitive to chloramphenicol.

In a next step, the host cell is transformed with a polynucleotide that allows for replacement of the gDNA with a gene of interest. By subsequent selection for chloramphenicol resistance, only the cells having the gDNA replaced with the gene of interest will survive, since the gRNA is no longer expressed, which makes the properly transformed host cells resistant to chloramphenicol.

As illustrated in the Examples enclosed herein, the methods of the present invention are particularly suitable for one-step multi-insertions of one or more specific expression cassettes on separate *loci* on the chromosome of a host cell. The method of the invention provides host cells containing multiple expression cassettes, *i.e.,* multi-copy host cells, that are highly stabile due to the expression cassettes being inserted on separate *loci* on the chromosome. Such cells are highly warranted in industrial biotechnology as robust workhorses for production of polypeptides of interest.

Thus, in a first aspect, the present invention relates to a method for inserting at least one polynucleotide of interest into the genome of a host cell, the method comprising the steps of:

  a) providing a host cell comprising in its genome:

    i. a polynucleotide encoding a selectable marker comprising a target sequence flanked by a functional PAM sequence for a Class-II Cas9 protein;
    ii. at least one polynucleotide encoding a gRNA that is at least 80% complementary to and capable of hybridizing to the target sequence; and
    iii. a polynucleotide encoding a nuclease-null variant of a Class-II Cas9 protein capable of interaction with the gRNA and binding to the target sequence, whereby expression of the selectable marker is repressed;

  b) transforming said host cell with at least one polynucleotide of interest and capable of inactivating the at least one polynucleotide encoding the gRNA;
  c) selecting for the trait conferred by the selectable marker; and
  d) identifying a transformed host cell, wherein the at least one polynucleotide encoding the gRNA has been inactivated by the at least one polynucleotide of interest.

The host cell provided in step (a) of the method of the first aspect comprises at least one polynucleotide encoding a gRNA. Preferably, the number of polynucleotides encoding a gRNA is at least one, such as at least two, at least three, at least four, at least five, at least six, at least seven, at least eight, at least nine, at least ten, at least 15, at least 20, at least 25, or more.

The host cell is transformed in step (b) of the method of the first aspect with at least one polynucleotide of interest. Preferably, the number of polynucleotide of interest is at least one, such as at least two, at least three, at least four, at least five, at least six, at least seven, at least eight, at least nine, at least ten, at least 15, at least 20, at least 25, or more.

Preferably, the at least one polynucleotide of interest encodes a protein; more preferably, the polynucleotide of interest encodes an enzyme selected from the group consisting of hydrolase, isomerase, ligase, lyase, oxidoreductase, or transferase; more preferably an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phosphodiesterase, phytase, polyphenoloxidase, proteolytic enzyme, ribonuclease, transglutaminase, xylanase, and beta-xylosidase.

Preferably, the selectable marker is an antibiotic resistance gene conferring resistance to chloramphenicol, tetracycline, ampicillin, spectinoymycin, kanamycin, or neomycin; more preferably, the selectable marker is an antibiotic resistance gene conferring resistance to chloramphenicol.

Also preferably, the selectable marker is an antibiotica resistance gene selected from the group consisting of *cat, erm, tet, amp, spec, kana,* and *neo*; more preferably, the selectable marker is a *cat* gene.

Alternatively, and also preferably, the selectable marker is a gene conferring auxotrophy to the host cell. Preferably, the selectable marker is a conditionally essential gene selected from the group consisting of *dal, lysA, araA, galE, antK metC, xylA, gntP, glpD, glpF, glpK, glpP, lacA2, hisC, gapA,* and *aspB* gene. More preferably, the selectable marker is a *dal* gene.

There are many well-known ways to inactivate a gene, for example by mutating the gene through the introduction of a non-sense mutation or a frameshift mutation, or by partial or full deletion of the open reading frame, or by manipulation of one or more control sequence.

Accordingly, in a preferred embodiment of the first aspect, the at least one polynucleotide encoding a gRNA is inactivated by partial or full deletion of said polynucleotide.

In a preferred embodiment of the first aspect, the at least one polynucleotide encoding the gRNA has been partially or fully replaced in the genome of the host cell by the at least one polynucleotide of interest in step (d), thereby inactivating the at least one polynucleotide encoding the gRNA.

In a second aspect, the present invention relates to a method for inserting at least two different polynucleotides of interest into the genome of a host cell, the method comprising the steps of:

    a) providing a host cell comprising in its genome:

        i. at least two polynucleotides encoding at least two different selectable markers, each comprising a different target sequence flanked by a functional PAM sequence for a Class-II Cas9 protein;
        ii. at least two polynucleotides encoding at least two gRNAs that are at least 80% complementary to and capable of hybridizing to the at least two different target sequences;
        iii. a polynucleotide encoding a nuclease-null variant of a Class-II Cas9 protein capable of interacting with the at least two gRNAs and binding to the at least two different target sequences, whereby expression of the two different selectable markers is repressed;

    b) transforming said host cell with at least two different polynucleotides of interest, said polynucleotides being capable of inactivating the at least two polynucleotides encoding the at least two gRNAs; and
    c) selecting for the traits conferred by the at least two different selectable markers; and
    d) identifying a transformed host cell, wherein the at least two polynucleotides encoding the at least two gRNAs have been inactivated by the at least two different polynucleotides of interest.

The host cell provided in step (a) of the method of the second aspect comprises at least two polynucleotides encoding at least two different selectable markers and at least two polynucleotides encoding at least two gRNAs. Preferably, the number of polynucleotides encoding the at least two different selectable markers and the at least two gRNAs are, independently, at least two, such as at least three, at least four, at least five, at least six, at least seven, at least eight, at least nine, at least ten, at least 15, at least 20, at least 25, or more.

The host cell is transformed in step (b) of the method of the second aspect with at least two different polynucleotides of interest. Preferably, the number of different polynucleotides of interest is at least two, such as at least three, at least four, at least five, at least six, at least seven, at least eight, at least nine, at least ten, at least 15, at least 20, at least 25, or more.

Preferably, the at least two different polynucleotides of interest encode at least two proteins; more preferably, the at least two different polynucleotides of interest encode enzymes independently selected from the group consisting of hydrolase, isomerase, ligase, lyase, oxidoreductase, or transferase; more preferably an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phosphodiesterase, phytase, polyphenoloxidase, proteolytic enzyme, ribonuclease, transglutaminase, xylanase, and beta-xylosidase.

In a preferred embodiment of the second aspect, the at least two different selectable markers are, independently, selected from the group consisting of antibiotic resistance genes and genes conferring auxotrophy to the host cell;

preferably, the at least two different selectable markers are, independently, selected from the group of genes consisting of *cat, erm, tet, amp, spec, kana, neo, dal, lysA, araA, galE, antK metC, xylA, gntP, glpD, glpF, glpK, glpP, lacA2, hisC, gapA,* and *aspB.*

In a preferred embodiment of the second aspect, the at least two polynucleotides encoding the at least two gRNAs are inactivated by partial or full deletion of said polynucleotides.

In a preferred embodiment of the second aspect, the at least two polynucleotides encoding the at least two gRNAs have been partially or fully replaced in the genome of the host cell by the at least two different polynucleotides of interest in step (d), thereby inactivating the at least two polynucleotides encoding the at least two gRNAs.

**Polynucleotides**

The present invention also relates to polynucleotides of the invention, including polynucleotides of interest as a well as polynucleotides encoding selectable markers, gRNAs, and nuclease-null variants of a Class-II Cas9 protein. In an embodiment, such polynucleotides have been isolated.

The techniques used to isolate or clone a polynucleotide are known in the art and include isolation from genomic DNA or cDNA, or a combination thereof. The cloning of the polynucleotides from genomic DNA can be affected, *e.g.,* by using the well-known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used.

**Nucleic Acid Constructs**

The present invention also relates to nucleic acid constructs comprising a polynucleotide of the present invention operably linked to one or more control sequences that direct the expression of the coding sequence in a suitable host cell under conditions compatible with the control sequences.

The polynucleotides may be manipulated in a variety of ways to provide for their expression. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including variant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and variant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide. Any terminator that is functional in the host cell may be used in the present invention.

Preferred terminators for bacterial host cells are obtained from the genes for *Bacillus clausii* alkaline protease (*aprH*), *Bacillus licheniformis* alpha-amylase (amyL), and *Escherichia coli* ribosomal RNA (*rrnB*).

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.*, 1992, *supra*.

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA*. Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus*

*oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polynucleotides relative to the growth of the host cell. Examples of regulatory sequences are those that cause expression of the polynucleotide to be turned on or off in response to a chemical or physical stimulus, including the presence of a regulatory compound. Regulatory sequences in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide would be operably linked to the regulatory sequence.

**Expression Vectors**

The present invention also relates to recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.*, a vector that exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, markers that confer auxotrophy for amino acids or other metabolites, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosylaminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus* bar gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is an *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra).*

**Host Cells**

The present invention also relates to recombinant host cells comprising polynucleotided of the present invention operably linked to one or more control sequences that direct expression of the polynucleotides of the invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication.

The host cell may be any useful cell, *e.g.*, a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus altitudinis, Bacillus amyloliquefaciens, B. amyloliquefaciens* subsp. *plantarum, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus methylotrophicus, Bacillus pumilus, Bacillus safensis, Bacillus stearothermophilus, Bacillus subtilis,* and *Bacillus thuringiensis* cells.

The bacterial host cell may also be any Streptococcus cell including, but not limited to, Streptococcus equisimilis, Streptococcus pyogenes, Streptococcus uberis, and Streptococcus equi subsp. Zooepidemicus cells.

The bacterial host cell may also be any Streptomyces cell including, but not limited to, Streptomyces achromogenes, Streptomyces avermitilis, Streptomyces coelicolor, Streptomyces griseus, and Streptomyces lividans cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (*Endomycetales*), basidiosporogenous yeast, and yeast belonging to the *Fungi Imperfecti* (*Blastomycetes*). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, *In* Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

**Nuclease-null variant of a Class-II Cas9 protein**

Several Class-II Cas9 analogues or homologues are known and more are being discovered as the scientific interest has surged over the last few years; a review is provided in Makarova et al., 20015, An updated evolutionary classification of CRISPR-Cas systems, Nature 13: 722-736.

The Cas9 protein of *Streptococcus pyogenes* (SEQ ID NO: 2) is a model Class-II Cas9 protein and it is to-date the best characterized. A variant of this protein was developed which has only one active nuclease domain (as opposed to the two active domains in the wildtype protein) by substituting a single amino acid, aspartic acid for alanine, in position 10: D10A. Another variant of this protein was developed which has only one active nuclease domain (as opposed to the two active domains in the wildtype protein) by substituting a single amino acid, histidine for alanine, in position 840: H840A. The doubly substituted (D10A, H840A) variant is a nuclease-null variant. It is expected that other Class-II Cas9 enzymes may be modified similarly.

Accordingly, in a preferred embodiment, the nuclease-null variant of the Class-II Cas9 protein comprises a substitution in the amino acid position corresponding to position 10 in the S. *pyogenes* Cas9 amino acid sequence; preferably the nuclease-null variant of the Class-II Cas9 protein comprises a substitution of aspartic acid for alanine, D10A, in the S. *pyogenes* Cas9 amino acid sequence. It is also preferred that the nuclease-null variant of the Class-II Cas9 protein comprises a substitution in the amino acid position corresponding to position 840 in the S. *pyogenes* Cas9 amino acid sequence; preferably the nuclease-null variant of the Class-II Cas9 protein comprises a substitution of histidine for alanine, H840A, in the S. *pyogenes* Cas9 amino acid sequence.

It is contemplated that nuclease-null variants of other endonucleases such as Cpf1 (Zetsche et al., Cell 2015, 163, pp. 759-771) and MADzymes such MAD7 released by Inscripta, Inc. (www.inscripta.com, formerly Muse Bio) may also be useful for counterselection purposes as described herein. Accordingly, such nuclease-null variants are within the scope of the present invention.

**Guide-RNA**

The gRNA in CRISPR-Cas9 genome editing constitutes the re-programmable part that makes the system so versatile. In the natural S. *pyogenes* system, the gRNA is actually a complex of two RNA polynucleotides, a first crRNA containing about 20 nucleotides that determine the specificity of the Cas9 protein and the tracr RNA which hybridizes to the crRNA to form an RNA complex that interacts with Cas9 (see Jinek et al., 2012, A programmable dual-RNA-guided DNA endonuclease in adaptive bacterial immunity, Science 337: 816-821). The terms crRNA and tracrRNA are used interchangeably with the terms tracr-mate RNA and tracr RNA herein.

Since the discovery of the CRISPR-Cas9 system single polynucleotide gRNAs have been developed and successfully applied just as effectively as the natural two part gRNA complex.

In a preferred embodiment, the single gRNA or RNA complex comprises a first RNA comprising 20 or more nucleotides that are at least 85% complementary to and capable of hybridizing to the one or more genome target sequence; preferably the 20 or more nucleotides are at least 90%, 95%, 97%, 98%, 99% or even 100% complementary to and capable of hybridizing to the one or more genome target sequence.

In another preferred embodiment, the host cell comprises a single gRNA comprising the first and second RNAs in the form of a single polynucleotide and wherein the tracr mate sequence and the tracr sequence form a stem-loop structure when hybridized with each other.

In order for a Cas9-gRNA complex to be capable of hybridizing with a given target sequence, the target sequence should be flanked by a functional PAM sequence for a Class-II Cas9 protein. For an overview of PAM sequences, see, for example, Shah et al, 2013, Protospacer recognition motifs, RNA Biol. 10(5): 891-899.

**Examples**

**Materials and methods**

Chemicals used as buffers and substrates were commercial products of at least reagent grade.

PCR amplifications were performed using standard textbook procedures, employing a commercial thermocycler and either Ready-To-Go PCR beads, Phusion polymerase, or RED-TAQ polymerase from commercial suppliers.

LB agar: See EP 0 506 780.

LBPSG agar plates contains LB agar supplemented with phosphate (0.01 M K3PO4), glucose (0.4 %), and starch (0.5 %); See EP 0 805 867 B1.

TY (liquid broth medium; See WO 1994/14968, p16.

Oligonucleotide primers were obtained from DNA technology, Aarhus, Denmark. DNA manipulations (plasmid and genomic DNA preparation, restriction digestion, purification, ligation, DNA sequencing) was performed using standard textbook procedures with commercially available kits and reagents.

Ligation mixtures were in some cases amplified in an isothermal rolling circle amplification reaction, using the TempliPhi kit from GE Healthcare.

DNA was introduced into B. *subtilis* rendered naturally competent, either using a two step procedure (Yasbin et al., 1975, J. Bacteriol. 121: 296-304.), or a one step procedure, in which cell material from an agar plate was resuspended in Spizisen 1 medium (WO 2014/052630), 12 ml shaken at 200 rpm for appr. 4 hours at 37 oC, DNA added to 400 microliter aliquots, and these further shaken 150 rpm for 1 hour at the desired temperature before plating on selective agar plates.

DNA was introduced into *B. licheniformis* by conjugation from *B. subtilis,* essentially as prevously described (EP 2 029 732 B1), using a modified B. *subtilis* donor strain PP3724, containing pLS20, wherein the methylase gene M.bli1904II (US20130177942) is expressed from a triple promoter at the *amyE* locus, the pBC16-derived orf beta and the B. *subtilis comS* gene (and a kanamycin resistance gene) are expressed from a triple promoter at the *alr* locus (making the strain D-alanine requiring), and the B. *subtilis comS* gene (and a *cat* gene) are expressed from a triple promoter at the *pel* locus.

*B. subtilis* JA1343: JA1343 is a sporulation negative derivative of PL1801 (WO 2005/042750). Part of the gene *spollAC* has been deleted to obtain the sporulation negative phenotype.

All of the constructions described in the examples were assembled from synthetic DNA fragments ordered from GeneArt - ThermoFisher Scientific. The fragments were assembled by sequence overlap extension (SOE) as described in the examples.

The temperature-sensitive plasmids used in this patent was incorporated into the genome of B. *licheniformis* by chromosomal integration and excision according to the method previously described (U.S. Patent No. 5,843,720). *B. licheniformis* transformants containing plasmids were grown on LBPG selective medium with erythromycin at 50°C to force integration of the vector at identical sequences to the chromosome. Desired integrants were chosen based on their ability to grow on LBPG + erythromycin selective medium at 50°C. Integrants were then grown without selection in LBPG medium at 37°C to allow excision of the integrated plasmid. Cells were plated on LBPG plates and screened for erythromycin-sensitivity. The sensitive clones were checked for correct integration of the desired construct.

Genomic DNA was prepared from several erythromycin sensitive isolates above accordingly to the method previously described (Pitcher et. al, *supra)* or by using the commercial available QIAamp DNA Blood Kit from Qiagen.

**Strains**

PP3724: Containing pLS20, wherein the methylase gene M.bli1904II (US 20130177942) is expressed from a triple promoter at the *amyE* locus, the pBC16-derived orf beta and the B. *subtilis comS* gene (and a kanamycin resistance gene) are expressed from a triple promoter at the *alr* locus (making the strain D-alanine requiring), and the B. *subtilis comS* gene (and a *cat* gene) are expressed from a triple promoter at the *pel* locus.

JA1622: This strain is the B. *subtilis* 168 derivative JA578 described in WO 2002/00907 with a disrupted *spollAC* gene (*sigF*). The genotype is: *amyE::repF* (pE194), *spollAC.*

SJ1904: This strain is a B. *licheniformis* strain described in WO 2008/066931. The gene encoding the alkaline protease (*aprL*) is inactivated.

PP3811: A derivative of B. *licheniformis* strain SJ1904, where the alkaline protease gene *aprL,* metalloprotease *mprL,* and the *spollAC* gene is inactivated.

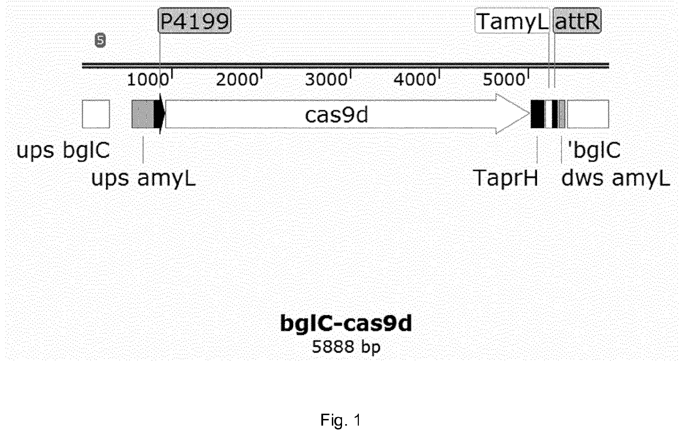

PP3811-cas9d: This strain is the B. *licheniformis* strain PP3811 where the cas9d gene is inserted at the *bglC* locus. The final insert has the cas9d gene transcribed from the *PamyL* promoter variant described in WO 1993/010249. The final sequence on the chromosome after integration is described in Fig. 1 and SEQ ID NO:3.

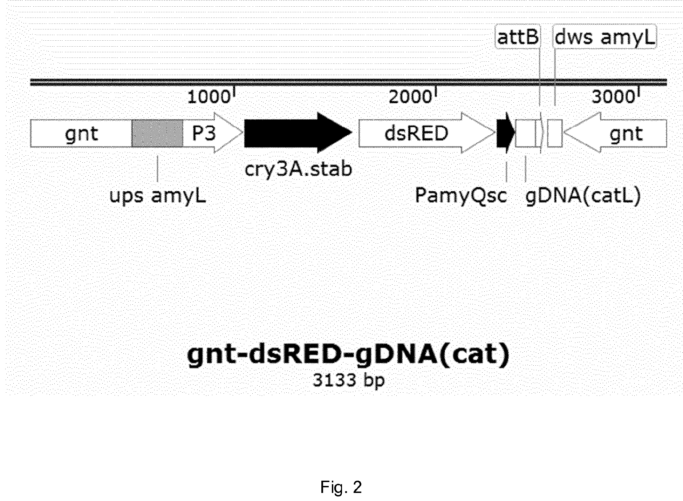

PP3811-gDNA1: This strain is the B. *licheniformis* strain PP3811-cas9d where the *dsRED* gene and a gDNA(cat) transcribing the gRNA(cat) directed against the *catL* gene in B. *licheniformis* is inserted into the *gnt* locus. Further downstream of gDNA the *attB* site from phage TP901-1 is positioned (WO 2006/042548). The *dsRED* gene is expressed from the triple promoter described in WO 1999/043835. The final sequence on the chromosome after integration is described in Fig. 2 and SEQ ID NO:4.

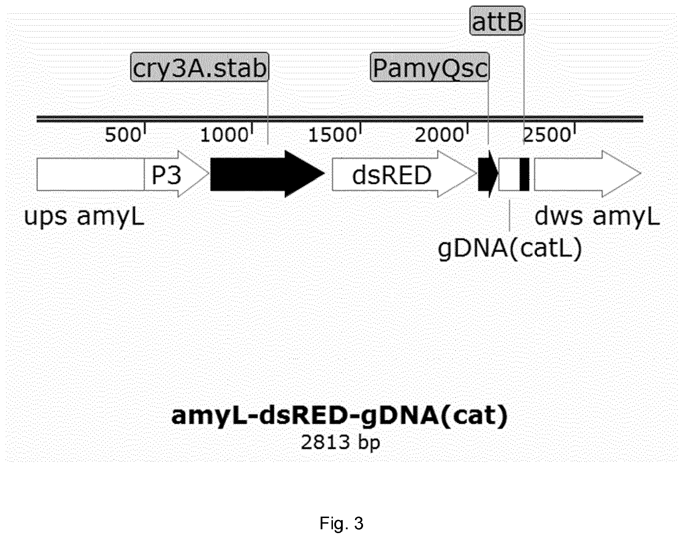

PP3811-gDNA2: This strain is the B. *licheniformis* strain PP3811-gDNA1 where the *dsRED* gene and a gDNA(cat) transcribing the gRNA(cat) directed against the *catL* gene in B. *licheniformis* is inserted into the *amyL* locus. Further downstream of gDNA the *attB* site is positioned (see above). The final sequence on the chromosome after integration is described in Fig. 3 and SEQ ID NO:5.

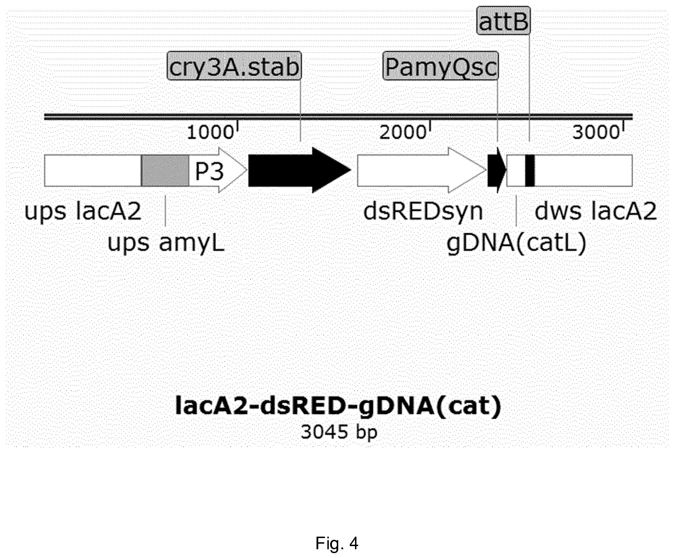

PP3811-gDNA3: This strain is the B. *licheniformis* PP3811-gDNA2 where the *dsRED* gene and a gDNA(cat) transcribing the gRNA(cat) directed against the *catL* gene in B. *licheniformis* is inserted into the *lacA2* locus. Further downstream of gDNA the *attB* site is positioned (see above). The final sequence on the chromosome after integration is described in Fig. 4 and SEQ ID NO:6.

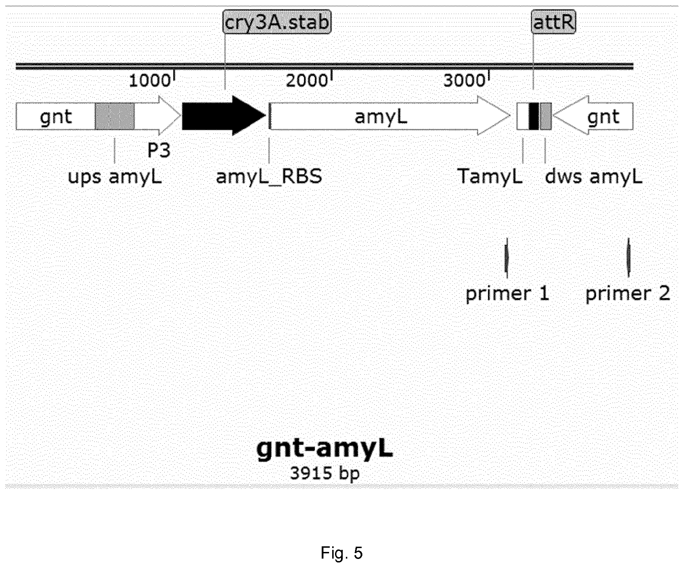

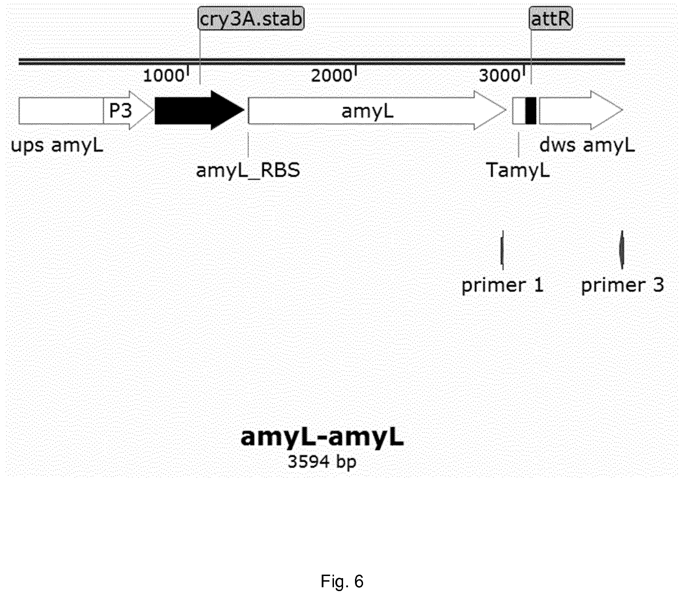

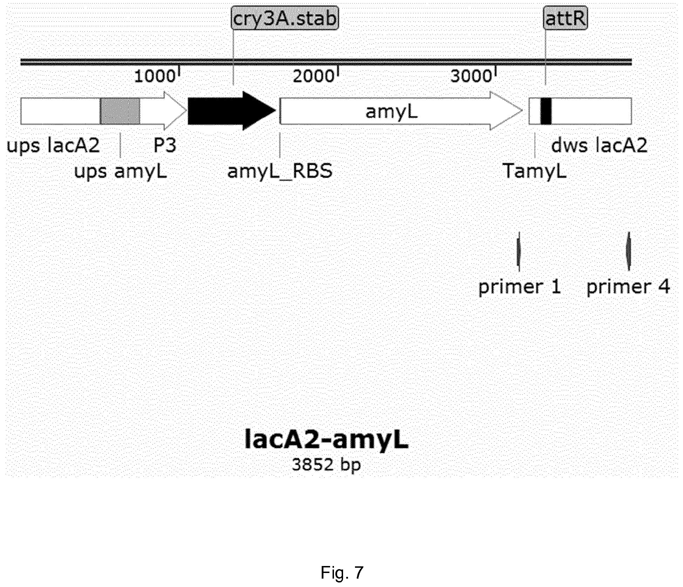

PP3811-amyL3: This is the B. *licheniformis* strain PP3811-gDNA3 where the three copies of *dsRED* gene and *gDNA(cat)* is replaced with three copies of the *amyL* gene encoding the alpha-amylase from B. *licheniformis.* The final sequence of the three loci of the chromosome after replacement is described in Figures 5-7 and SEQ ID NO:7-9.

PP3724-pPPamyL-attP: This strain is the conjugation donor strain PP3724 holding the plasmid pPPamyL-attP.

**Plasmids**

pC194: Plasmid isolated from *Staphylococcus aureus* (Horinouchi and Weisblum, 1982).

pE194: Plasmid isolated from *S. aureus* (Horinouchi and Weisblum, 1982).

pUB110: Plasmid isolated from *S. aureus* (McKenzie et al., 1986)

pPPamyL-attP: Plasmid constructed for this invention in Example 6. The plasmid was made by assembly of synthetic sequences to generate a vector holding the: (1) *amyL* gene encoding the alpha-amylase from B. *licheniformis* preceded by the *cry3A* stabilizer for integration (2) the *attP* and the integrase (int) from TP901-1 described in WO 2006/042548. The integrase promote integration between the *attP* site on the plasmid and the *attB* site on the chromosome of the B. *licheniformis* host.

**Sequences**

| SEQ ID NO | Name |
| --- | --- |
| 1 | *S. pyogenes* Cas9 DNA sequence |
| 2 | S. *pyogenes* Cas9 amino acid sequence |
| 3 | Sequence of bglC-cas9d *locus* |
| 4 | Sequence of gnt *locus* of PP3811-gDNA3: gnt-dsRED-gDNA(cat) |
| 5 | Sequence of amyL *locus* of PP3811-gDNA3: amyL-dsRED-gDNA(cat) |
| 6 | Sequence of lacA2 *locus* of PP3811-gDNA3: lacA2-dsRED-gDNA(cat) |
| 7 | Sequence of the gnt *locus* after integration of amyL in PP3811-amyL3 |
| 8 | Sequence of the amyL *locus* after re-integration of amyL in PP381 1-amyL3 |
| 9 | Sequence of the lacA2 *locus* after integration of amyL in PP3811-amyL3 |
| 10 | Sequence of the plasmid pPPamyL-attP |
| 11 | Primer 1 (see Fig. 5-7) |
| 12 | Primer 2 (see Fig. 5-7) |
| 13 | Primer 3 (see Fig. 5-7) |
| 14 | Primer 4 (see Fig. 5-7) |
| 15 | Sequence of Bglll-Mlul fragment |

**Example 1. Chromosomal integration of *cas9d* into the *bglC* locus of *B. licheniformis***

An expression cassette was inserted at the *bglC* locus where the cas9d gene encoding the inactive variant of Cas9 (Cas9d) is expressed from the *amyL* promoter (P4199) earlier described in WO 1993/010249. The DNA for integration was ordered as synthetic DNA (GeneArt - ThermoFisher Scientific) and cloned into integration vectors as earlier described in WO 2006/042548. The final map of the *bglC* locus is shown in Fig. 1. The nucleotide sequence of the *locus* can be found in SEQ ID NO:3.

The condition for the PCR amplifications is as follows: The respective DNA fragments were amplified by PCR using the Phusion Hot Start DNA Polymerase system (Thermo Scientific). The PCR amplification reaction mixture contained 1 ul (~0,1 ug) of template DNA, 2 ul of sense primer (20 pmol/ul), 2 ul of anti-sense primer (20 pmol/ul), 10 ul of 5X PCR buffer with 7.5 mM MgCl$_2$, 8 ul of dNTP mix (1,25 mM each), 37 $\mu$l water, and 0.5 ul (2 U/ul) DNA polymerase mix. A thermocycler was used to amplify the fragment. The PCR products were purified from a 1.2% agarose gel with 1x TBE buffer using the Qiagen QIAquick Gel Extraction Kit (Qiagen, Inc., Valencia, CA) according to the manufacturer's instructions.

The PCR products were used in a subsequent PCR reactions to create a single plasmid using splice overlapping PCR (SOE) using the the Phusion Hot Start DNA Polymerase system (Thermo Scientific) as follows. The PCR amplification reaction mixture contained 50 ng of each of the two gel purified PCR products and the synthetic fragment and a thermocycler was used to assemble and amplify the plasmid. The resulting SOE product was used directly for transformation to B. subtilis host JA1622 to establish the plasmid. The plasmid was transferred by competence to the donor strain PP3724.

The strain B. *licheniformis* strain was transformed with the plasmid described above and integrated and excised according to the procedure described above in the Materials and Methods section. By this procedure the *bglC* locus on the chromosome is replaced with the cloned construct delivered by the plasmid. The final strain has replaced the *bglC* locus with the construct shown in Fig. 1 and the plasmid is lost at restrictive temperature at 50°C.

The final construct has the *cas9d* gene expressed from the *bglC* locus on the chromosome. The strain is named PP3811-cas9d.

**Example 2. Chromosomal integration of *dsRED*-gDNA(*cat*) into the *gnt* locus of *B. licheniformis***

An expression cassette was inserted at the *gnt* locus where the *dsRED* marker gene encoding the red fluorescent protein is expressed from the P3 promoter earlier described in WO 2005/098016. Downstream of the *dsRED* marker gene a gDNA sequence is expressed from the *amyQ* promoter from B. *amyloliquefaciens.* The gDNA transcribes a gRNA directed against the *cat* marker gene. The *cat* marker gene encodes an acetyl transferase from *B. licheniformis* which confer resistance to chloramphenicol. The chromosomal integration of DNA into B. *licheniformis* has been described in WO 2007/138049. The DNA for integration was ordered as synthetic DNA (GeneArt - ThermoFisher Scientific), assembled by SOE-PCR and cloned into temperature sensitive integration vectors based on pE194 as earlier described. The final map of the *gnt* locus is shown in Fig. 1. The nucleotide sequence of the locus can be found in SEQ ID NO:4.

The PCR products were made as described in Example 1 and used in a subsequent PCR reaction to create a single plasmid using splice overlapping PCR (SOE) using the Phusion Hot Start DNA Polymerase system (Thermo Scientific) as follows. The PCR amplification reaction mixture contained 50 ng of each of the two gel purified PCR products and the synthetic fragment and a thermocycler was used to assemble and amplify the integration plasmid. The resulting SOE product was used directly for transformation to B. *subtilis* host JA1622 to establish the integration plasmid. The plasmid is transferred to the donor strain PP3724 and used for conjugation. The plasmid is used to insert the *dsRED* gene and the gDNA(cat) at the *gnt* locus of B. *licheniformis* according to the procedure described in Example 1. The final strain is named PP3811-gDNA1.

**Example 3. Chromosomal integration of *dsRED*-gDNA(*cat*) into the *amyL* locus of *B. licheniformis***

An expression cassette identical to the one described in Example 2 was inserted at the *amyL* locus. The DNA for integration was ordered as synthetic DNA (GeneArt - ThermoFisher Scientific) assembled by SOE-PCR and cloned into temperature sensitive integration vectors based on pE194 as earlier described in WO 2006/042548. The final map of the *amyL* locus is shown in Fig. 3. The nucleotide sequence of the locus can be found in SEQ ID NO:5.

The PCR products were made as described in example 1 and used in a subsequent PCR reaction to create a single plasmid using splice overlapping PCR (SOE) using the Phusion Hot Start DNA Polymerase system (Thermo Scientific) as follows. The PCR amplification reaction mixture contained 50 ng of each of the two gel purified PCR products and the synthetic fragment and a thermocycler was used to assemble and amplify the integration plasmid. The resulting SOE product was used directly for transformation to B. *subtilis* host JA1622 to establish the integration plasmid. This plasmid is used to insert the *dsRED* gene and the gDNA(cat) at the *amyL* locus of B. *licheniformis* as described above in Example 1. The final strain is named PP3811-gDNA2.

**Example 4. Chromosomal integration of *dsRED*-gDNA(*cat*) into the *lacA2* locus of *B. licheniformis***

An expression cassette was inserted at the *lacA2* locus almost identical to the one described in Examples 2 and 3. The only difference is an alternative synthetic sequence of the *dsRED* gene (dsREDsyn). This gene variant still encodes the same fluorescent protein. The DNA for integration was ordered as synthetic DNA (GeneArt - ThermoFisher Scientific) and cloned into integration vectors earlier described in WO 2006/042548. The final map of the *amyL* locus is shown in Fig. 4. The nucleotide sequence of the locus can be found in SEQ ID NO:6.

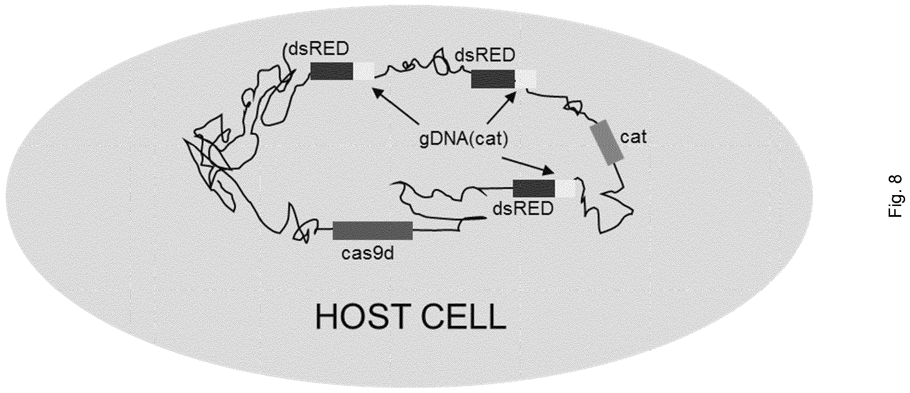

The PCR products were made as described in Example 1 and used in a subsequent PCR reaction to create a single plasmid using splice overlapping PCR (SOE) using the Phusion Hot Start DNA Polymerase system (Thermo Scientific) as follows. The PCR amplification reaction mixture contained 50 ng of each of the two gel purified PCR products and the synthetic fragment and a thermocycler was used to assemble and amplify the integration plasmid. The resulting SOE product was used directly for transformation to *B. subtilis* host JA1622 to establish the integration plasmid. This plasmid is used to insert the *dsRED* gene and the gDNA(cat) at the *lacA2* locus of B. *licheniformis* as described above in Example 1. The final strain is named PP3811-gDNA3 which has three copies of the *dsRED* gene and the gDNA(catL) cassette and the Cas9d expressed from the *bglC* locus (Fig. 8).

## Example 5. Construction of the plasmid pPPamyL-attP

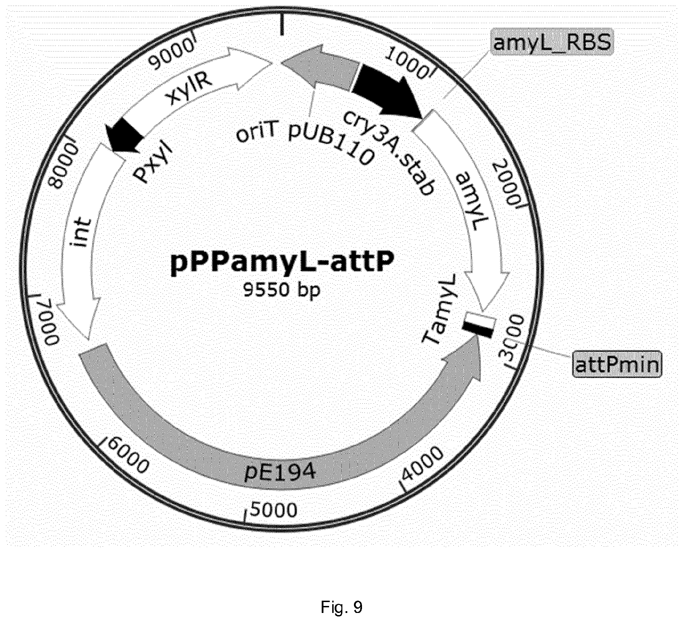

The plasmid pPPamyL-attP was assembled from DNA sequences ordered from GeneArt. The entire plasmid and the annotations is depicted in Fig. 9. The nucleotide sequence of the plasmid can be found in SEQ ID NO:10.

The condition for the PCR amplifications is as described in Example 1. The purified PCR products were used in a subsequent PCR reaction to create a single plasmid using splice overlapping PCR (SOE) using the the Phusion Hot Start DNA Polymerase system (Thermo Scientific) as follows. The PCR amplification reaction mixture contained 50 ng of each of the six gel purified PCR products and a thermocycler was used to assemble and amplify the plasmid of 9550 bp (Fig. 1). The resulting SOE product was used directly for transformation to B. subtilis host JA1622 to establish the plasmid pPPamyL-attP. The plasmid is used in Example 6 for transformation of the host strain described in Example 4, PP64.

The plasmid encodes the amylase gene *amyL* from B. *licheniformis* flanked upstream by the *cry3A* stabilizer region and the *attP* phage integration site.

The integration of the *amyL* into the chromosome will take place between the *cry3A* stabilizer regions present in the host strain PP3811-gDNA3 and on the plasmid and the *attB* and *attP* sites on the chromosome and plasmid respectively.

## Example 6. Selection for a three-copy integration of the amylase gene *amy*L

The plasmid pPPamyL-attP described in Example 5 is transformed into the B. *licheniformis* strain PP3811-gDNA3 to select for on-step integration of the *amyL* expression cassette in the three different loci, gnt:dsRED-gDNA(cat), amyL:dsRED-gDNA(cat), and lacA2:dsRED-gDNA(cat). In this step the gDNA(cat) and the *dsRED* gene is replaced by the *amyL* expression cassette. The replacement is potentiated by the recombination between flanking regions on the gDNA loci on the chromosome and the introduced plasmid. Upstream by the identical *cry3A* stabilizer regions present on the chromosome of the host strain PP3811-gDNA3 and on the plasmid pPPamyL-attP. Downstream by the *attB* and *attP* sites on the chromosome and plasmid respectively.

After plasmid transformation of the PP3811-gDNA3 the cells are plated for three days on LBPG plates with 1 ug/ml of erythromycin at 34°C to allow amplification and recombination events to occur between the chromosome and the plasmid at permissive temperature. The colonies are washed of in 200 ul TY and 50 ul is transferred to 5 ml of liquid cultures in TY and incubated at 200 rpm at 34 °C for 24 hours. The culture is streaked on LBPG plates with 6 ug/ml chloramphenicol (cam) to select for strains where all three gDNA(*cat*) loci are replaced with the amyL expression cassette.

Ten different colonies from the cam plates are re-streaked and tested for *amyL* integration in all three loci. All ten colonies show the expected bands on an agarose gel.

All three loci were efficiently replaced and the *amyL* expression cassette was inserted as expected. Fig. 5-7 show all of the loci after replacement and the primers used for verification of the insert. The strain is named PP3811-3amyL

The chloramphenicol resistant clones all has amylase activity shown by plating on LBPG plates supplemented with starch. All colonies show big halos on plates supplemented with starch verifying expression of amylase.

This example show that the gDNA(cat) in combination with Cas9d can very efficiently be employed as a tool to select for integration of at least three copies of an expression cassette on the chromosome of *B. licheniformis.*

## Example 7. Host cell construction for selection of three-copy integration of DNA using the flp/FRT technology.

The B. *licheniformis* strain PP3811-gDNA3 (Example 4) was used as basis for construction of a strain, where the integration of three copies of a gene of interest by way of the flp/FRT technology (WO 2018/077796) could be selected for.

In Example 6, an *amyL* integration cassette was inserted in each of the three different loci available for strain PP3811-gDNA3, i.e. gnt:dsRED-gDNA(cat), amyL:dsRED-gDNA(cat), and lacA2:dsRED-gDNA(cat).

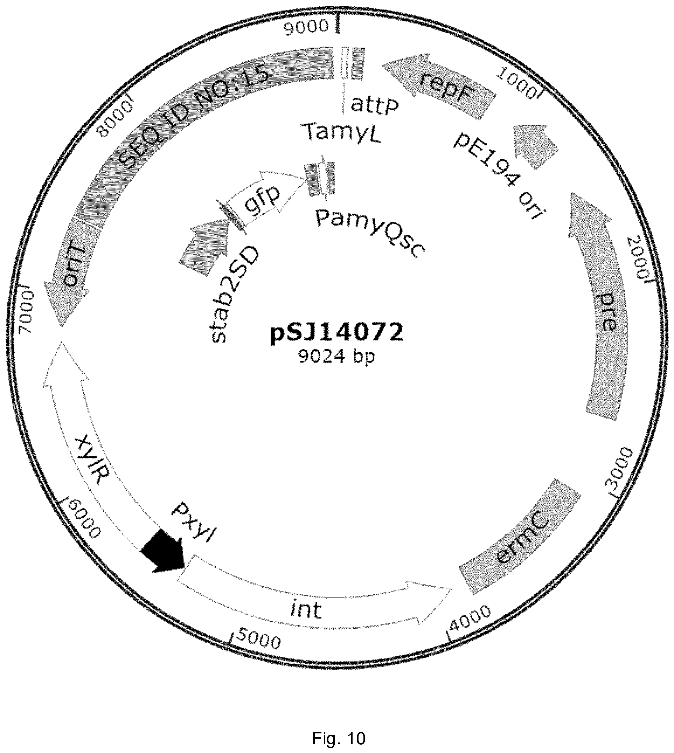

In the present example, the same procedure as used for *amyL* integration, except for the CamR selection, was used to integrate a segment consisting of FRT-F, a green fluorescent protein (GFP) encoding region, a gDNA sequence transcribing a gRNA directed against the *cat* marker gene, expressed from the *amyQ* promoter from *B. amyloliquefaciens*,

and FRT-F3 into PP3811-gDNA3. The DNA for integration was ordered as synthetic DNA (GeneArt - ThermoFisher Scientific), and received in a vector kept as pSJ13864. A 1.6 kb BglII-MluI fragment was excised from pSJ13864 and ligated to the 7.4 kb BglII-MluI fragment of pPP3676, resulting in pSJ14072 (Fig. 10). The 1.6 kb BglII-MluI fragment has the DNA sequence given in SEQ ID NO:15.

pSJ14072 was introduced into the conjugative donor strain PP3724, and the resulting pool of transformants was used as donor in conjugation to PP3811-gDNA3. Recombination between plasmid and chromosome took place as described in example 6. Strains resulting from the desired integration in all three loci were isolated as green fluorescent colonies, easily visible against the red fluorescent colonies of the parent organism. All cells were still chloramphenicol sensitive, as the gDNA transcribing the gRNA directed against *cat* was still present at all 3 loci. Such a host cell was kept as SJ14111.

**Example 8. Isolation of strains selected to contain three copies of the amylase gene *amyL* using the flp/FRT technology.**

To construct an alpha-amylase integration vector for the flp/FRT system, the *amyL* coding region was amplified from *Bacillus licheniformis* using primers #B692 and #B693 and cloned into pSJ13654 as earlier described (WO 2018/077796), the desired recombinant plasmid was obtained (pSJ13835), introduced into a *Bacillus subtilis* conjugative donor strain, and a transformant kept as SJ13837. The integration vector thus carries a segment consisting of FRT-F, *amyL,* and FRT-F3.

SJ13837 was used as donor in conjugation into SJ14111. After growth of the mixed donor/recipient culture on solid media, a replica was made onto media with erythromycin (2 ug/ml), the plates incubated at 33 °C for 4 days, whereafter a streak of mixed transconjugants (using a 10 ul inoculation loop) were inoculated into liquid medium (TY) either with or without chloramphenicol (6 ug/ml), and incubated with shaking at 37 °C. Next day the culture without chloramphenicol showed strong green fluorescence, while the culture with chloramphenicol showed no fluorescence. This is in agreement with the notion that chloramphenicol resistance is obtained if something (in this case *amyL*) replaces the GFP+gDNA(*cat*) in the chromosome. Cultures were used to inoculate new cultures, this time with or without 10 ug/ml chloramphenicol, and again the cultures with chloramphenicol showed no fluorescence. Upon plating, a significant number of non-fluorescing colonies were obtained. Four such colonies were confirmed to be chloramphenicol resistant and erythromycin sensitive and, when analyzed by PCR amplification across each of the three integration loci, all were confirmed to contain the insertion of the FRT-flanked *amyL* construct.

**Claims**

1.  A method for inserting at least one polynucleotide of interest into the genome of a host cell, the method comprising the steps of:

    a) providing a host cell comprising in its genome:

       i. a polynucleotide encoding a selectable marker comprising a target sequence flanked by a functional PAM sequence for a Class-II Cas9 protein;
       ii. at least one polynucleotide encoding a gRNA that is at least 80% complementary to and capable of hybridizing to the target sequence; and
       iii. a polynucleotide encoding a nuclease-null variant of a Class-II Cas9 protein capable of interaction with the gRNA and binding to the target sequence, whereby expression of the selectable marker is repressed;

    b) transforming said host cell with at least one polynucleotide of interest and capable of inactivating the at least one polynucleotide encoding the gRNA;
    c) selecting for the trait conferred by the selectable marker; and
    d) identifying a transformed host cell, wherein the at least one polynucleotide encoding the gRNA has been inactivated by the at least one polynucleotide of interest.

2.  A method for inserting at least two different polynucleotides of interest into the genome of a host cell, the method comprising the steps of:

    a) providing a host cell comprising in its genome:

       i. at least two polynucleotides encoding at least two different selectable markers, each comprising a different target sequence flanked by a functional PAM sequence for a Class-II Cas9 protein;

ii. at least two polynucleotides encoding at least two gRNAs that are at least 80% complementary to and capable of hybridizing to the at least two different target sequences;

iii. a polynucleotide encoding a nuclease-null variant of a Class-II Cas9 protein capable of interacting with the at least two gRNAs and binding to the at least two different target sequences, whereby expression of the two different selectable markers is repressed;

b) transforming said host cell with at least two different polynucleotides of interest, said polynucleotides being capable of inactivating the at least two polynucleotides encoding the at least two gRNAs; and

c) selecting for the traits conferred by the at least two different selectable markers; and

d) identifying a transformed host cell, wherein the at least two polynucleotides encoding the at least two gRNAs have been inactivated by the at least two different polynucleotides of interest.

3. The method according to any of the preceding claims, wherein the at least one polynucleotide or the at least two polynucleotides of interest encode(s) a protein; preferably, the at least one polynucleotide or the at least two polynucleotides of interest encode(s) an enzyme independently selected from the group consisting of hydrolase, isomerase, ligase, lyase, oxidoreductase, or transferase; more preferably an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phosphodiesterase, phytase, polyphenoloxidase, proteolytic enzyme, ribonuclease, transglutaminase, xylanase, and beta-xylosidase.

4. The method according to any of the preceding claims, wherein the host cell is a Gram-positive host cell; preferably, the host cell is selected from the group consisting of *Bacillus, Streptomyces, Streptococcus,* and *Lactobacillus* host cell; more preferably, the host cell is selected from the group consisting of *Bacillus alkalophilus, Bacillus altitudinis, Bacillus amyloliquefaciens, B. amyloliquefaciens* subsp. *plantarum, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus methylotrophicus, Bacillus pumilus, Bacillus safensis, Bacillus stearothermophilus, Bacillus subtilis,* and *Bacillus thuringiensis* cells; most preferably, the prokaryotic host cell is a *Bacillus licheniformis* host cell.

5. The method according to any of claims 1-3, wherein the host cell is a fungal host cell selected from the group of consisting of *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* and *Trichoderma* cell; preferably the fungal host cell is selected from the group consisting of *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* and *Trichoderma viride* cell.

6. The method according to any of claims 1-3, wherein the host cell is a yeast host cell selected from the group consisting of *Candida, Hansenula, Kluyveromyces, Pichia, Saccharomyces, Schizosaccharomyces,* and *Yarrowia* cell; preferably the host cell is selected from the group consisting of *Kluyveromyces lactis, Pichia pastoris, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* and *Yarrowia lipolytica* cell.

7. The method according to any of the preceding claims, wherein the selectable marker or the at least two different selectable markers are, independently, a positive selection marker, a negative selection marker, a bidirectional marker, or a conditionally essential gene.

8. The method according to any of the preceding claims, wherein the selectable marker or the at least two different selectable markers are, independently selected from the group of genes consisting of *cat, erm, tet, amp, spec, kana, neo, dal, lysA, araA, galE, antK, metC, xylA, gntP, glpD, glpF, glpK, glpP, lacA2, hisC, gapA,* and *aspB.*

9. The method according to any of the preceding claims, wherein the gRNA or the at least two gRNAs comprise a first RNA comprising 20 or more nucleotides that are at least 85% complementary to and capable of hybridizing to the polynucleotide(s) encoding the selectable marker(s); preferably, the 20 or more nucleotides are at least 90%, 95%, 97%, 98%, 99% or even 100% complementary to and capable of hybridizing to the polynucleotide(s) encoding the selectable marker(s).

10. The method according to any of the preceding claims, wherein the nuclease-null variant of a Class-II Cas9 protein comprises an alteration of an amino acid corresponding to position 10 and position 840 of SEQ ID NO:2; more preferably said variant comprises a substitution of aspartic acid for alanine, D10A, and a substitution of histidine for alanine, H840A.

11. The method according to any of the preceding claims, wherein the at least one polynucleotide encoding the gRNA or the at least two polynucleotides encoding the at least two gRNAs have been partially or fully replaced in the genome of the host cell by the at least one polynucleotide of interest or the at least two different polynucleotides of interest, thereby inactivating the at least one polynucleotide encoding the gRNA or the at least two polynucleotides encoding the at least two gRNAs.

**Patentansprüche**

1. Verfahren zum Insertieren von mindestens einem Polynukleotid von Interesse in das Genom einer Wirtszelle, wobei das Verfahren die Schritte umfasst:

    a) Bereitstellen einer Wirtszelle, die in ihrem Genom umfasst:

        i. ein Polynukleotid, das einen Selektionsmarker kodiert, der eine Zielsequenz umfasst, die von einer funktionellen PAM-Sequenz für ein Klasse-II-Cas9-Protein flankiert ist;
        ii. mindestens ein Polynukleotid, das eine gRNA kodiert, die mindestens 80% komplementär zu und fähig zum Hybridisieren mit der Zielsequenz ist; und
        iii. ein Polynukleotid, das eine Nuklease-Null-Variante eines Klasse-II-Cas9-Proteins kodiert, die zu Wechselwirkung mit der gRNA und Binden an die Zielsequenz fähig ist, wodurch eine Expression des Selektionsmarkers unterdrückt wird;

    b) Transformieren der Wirtszelle mit mindestens einem Polynukleotid von Interesse und fähig zum Inaktivieren des mindestens einen die gRNA kodierenden Polynukleotids;
    c) Selektieren auf das durch den Selektionsmarker verliehene Merkmal; und
    d) Identifizieren einer transformierten Wirtszelle, wobei das mindestens eine die gRNA kodierende Polynukleotid durch das mindestens eine Polynukleotid von Interesse inaktiviert wurde.

2. Verfahren zum Insertieren von mindestens zwei verschiedenen Polynukleotiden von Interesse in das Genom einer Wirtszelle, wobei das Verfahren die Schritte umfasst:

    a) Bereitstellen einer Wirtszelle, die in ihrem Genom umfasst:

        i. mindestens zwei Polynukleotide, die mindestens zwei verschiedene Selektionsmarker kodieren, die jeweils eine unterschiedliche Zielsequenz umfassen, die von einer funktionellen PAM-Sequenz für ein Klasse-II-Cas9-Protein flankiert ist;
        ii. mindestens zwei Polynukleotide, die mindestens zwei gRNAs kodieren, die mindestens 80% komplementär zu und fähig zum Hybridisieren mit den mindestens zwei verschiedenen Zielsequenzen sind;
        iii. ein Polynukleotid, das eine Nuklease-Null-Variante eines Klasse-II-Cas9-Proteins kodiert, das fähig zum Wechselwirken mit den mindestens zwei gRNAs und Binden an die mindestens zwei verschiedenen Zielsequenzen ist, wodurch eine Expression der zwei verschiedenen Selektionsmarker unterdrückt wird;

b) Transformieren der Wirtszelle mit mindestens zwei verschiedenen Polynukleotiden von Interesse, wobei die Polynukleotide fähig zum Inaktivieren der mindestens zwei Polynukleotide sind, die die mindestens zwei gRNAs kodieren; und

c) Selektieren auf die durch die mindestens zwei verschiedenen Selektionsmarker verliehenen Eigenschaften; und

d) Identifizieren einer transformierten Wirtszelle, wobei die mindestens zwei Polynukleotide, die die mindestens zwei gRNAs kodieren, durch die mindestens zwei verschiedenen Polynukleotide von Interesse inaktiviert wurden.

3. Verfahren nach einem beliebigen der voranstehenden Ansprüche, wobei das mindestens eine Polynukleotid oder die mindestens zwei Polynukleotide von Interesse ein Protein kodiert/kodieren; bevorzugt kodiert/kodieren das mindestens eine Polynukleotid oder die mindestens zwei Polynukleotide von Interesse ein Enzym, das unabhängig ausgewählt ist aus der Gruppe bestehend aus Hydrolase, Isomerase, Ligase, Lyase, Oxidoreduktase oder Transferase; stärker bevorzugt eine Aminopeptidase, Amylase, Carbohydrase, Carboxypeptidase, Katalase, Cellobiohydrolase, Cellulase, Chitinase, Cutinase, Cyclodextrin-Glycosyltransferase, Desoxyribonuclease, Endoglucanase, Esterase, alpha-Galactosidase, beta-Galactosidase, Glucoamylase, alpha-Glucosidase, beta-Glucosidase, Invertase, Laccase, Lipase, Mannosidase, Mutanase, Oxidase, pektinolytisches Enzym, Peroxidase, Phosphodiesterase, Phytase, Polyphenoloxidase, proteolytisches Enzym, Ribonuklease, Transglutaminase, Xylanase und beta-Xylosidase.

4. Verfahren nach einem beliebigen der voranstehenden Ansprüche, wobei die Wirtszelle eine grampositive Wirtszelle ist; bevorzugt ist die Wirtszelle ausgewählt aus der Gruppe bestehend aus einer *Bacillus-, Streptomyces-, Streptococcus-* und Lactobacillus-Wirtszelle; stärker bevorzugt ist die Wirtszelle ausgewählt aus der Gruppe bestehend aus *Bacillus alkalophilus-, Bacillus altitudinis-, Bacillus amyloliquefaciens-, B. amyloliquefaciens* subsp. *Plantarum-, Bacillus brevis-, Bacillus circulans-, Bacillus clausii-, Bacillus coagulans-, Bacillus firmus-, Bacillus lautus-, Bacillus lentus-, Bacillus licheniformis-, Bacillus megaterium-, Bacillus methylotrophicus-, Bacillus pumilus-, Bacillus safensis-, Bacillus stearothermophilus-, Bacillus subtilis-,* und *Bacillus thuringiensis*-Zellen; am stärksten bevorzugt ist die prokaryotische Wirtszelle eine *Bacillus* licheniformis-Wirtszelle.

5. Verfahren nach einem beliebigen der Ansprüche 1-3, wobei die Wirtszelle eine Pilzwirtszelle ist, ausgewählt aus der Gruppe bestehend aus einer *Acremonium-, Aspergillus-, Aureobasidium-, Bjerkandera-, Ceriporiopsis-, Chrysosporium-, Coprinus-, Coriolus-, Cryptococcus-, Filibasidium-, Fusarium-, Humicola-, Magnaporthe-, Mucor-, Myceliophthora-, Neocallimastix-, Neurospora-, Paecilomyces-, Penicillium-, Phanerochaete-, Phlebia-, Piromyces-, Pleurotus-, Schizophyllum-, Talaromyces-, Thermoascus-, Thielavia-, Tolypocladium-, Trametes-* und *Trichoderma*-Zelle; bevorzugt ist die Pilzwirtszelle ausgewählt aus der Gruppe bestehend aus einer *Aspergillus awamori-, Aspergillus foetidus-, Aspergillus fumigatus-, Aspergillus japonicus-, Aspergillus nidulans-, Aspergillus niger-, Aspergillus oryzae-, Bjerkandera adusta-, Ceriporiopsis aneirina-, Ceriporiopsis caregiea-, Ceriporiopsis gilvescens-, Ceriporiopsis pannocinta-, Ceriporiopsis rivulosa-, Ceriporiopsis subrufa-, Ceriporiopsis subvermispora-, Chrysosporium inops-, Chrysosporium keratinophilum-, Chrysosporium lucknowense-, Chrysosporium merdarium-, Chrysosporium pannicola-, Chrysosporium queenslandicum-, Chrysosporium tropicum-, Chrysosporium zonatum-, Coprinus cinereus-, Coriolus hirsutus-, Fusarium bactridioides-, Fusarium cerealis-, Fusarium crookwellense-, Fusarium culmorum-, Fusarium graminearum-, Fusarium graminum-, Fusarium heterosporum-, Fusarium negundi-, Fusarium oxysporum-, Fusarium reticulatum-, Fusarium roseum-, Fusarium sambucinum-, Fusarium sarcochroum-, Fusarium sporotrichioides-, Fusarium sulphureum-, Fusarium torulosum-, Fusarium trichothecioides-, Fusarium venenatum-, Humicola insolens-, Humicola lanuginosa-, Mucor miehei-, Myceliophthora thermophila-, Neurospora crassa-, Penicillium purpurogenum-, Phanerochaete chrysosporium-, Phlebia radiata-, Pleurotus eryngii-, Thielavia terrestris-, Trametes villosa-, Trametes versicolor-, Trichoderma harzianum-, Trichoderma koningii-, Trichoderma longibrachiatum-, Trichoderma reesei-* und *Trichoderma viride*-Zelle.

6. Verfahren nach einem beliebigen der Ansprüche 1-3, wobei die Wirtszelle eine Hefewirtszelle ist, ausgewählt aus der Gruppe bestehend aus einer *Candida-, Hansenula-, Kluyveromyces-, Pichia-, Saccharomyces-, Schizosaccharomyces-* und *Yarrowia*-Zelle; bevorzugt ist die Wirtszelle ausgewählt aus der Gruppe bestehend aus einer *Kluyveromyces lactis-, Pichia pastoris-, Saccharomyces carlsbergensis-, Saccharomyces cerevisiae-, Saccharomyces diastaticus-, Saccharomyces douglasii-, Saccharomyces kluyveri-, Saccharomyces norbensis-, Saccharomyces oviformis-* und *Yarrowia lipolytica*-Zelle.

7. Verfahren nach einem beliebigen der voranstehenden Ansprüche, wobei der Selektionsmarker oder die mindestens zwei verschiedenen Selektionsmarker unabhängig ein positiver Selektionsmarker, ein negativer Selektionsmarker, ein bidirektionaler Marker oder ein bedingt essentielles Gen sind.

8. Verfahren nach einem beliebigen der voranstehenden Ansprüche, wobei der Selektionsmarker oder die mindestens zwei verschiedenen Selektionsmarker unabhängig aus der Gruppe von Genen ausgewählt sind, die aus *cat, erm, tet, amp, spec, kana, neo, dal, lysA, araA, galE, antK, metC, xylA, gntP, glpD, glpF, glpK, glpP, lacA2, hisC, gapA* und *aspB* besteht.

9. Verfahren nach einem beliebigen der voranstehenden Ansprüche, wobei die gRNA oder die mindestens zwei gRNAs eine erste RNA umfassen, die 20 oder mehr Nukleotide umfasst, die mindestens 85% komplementär zu und fähig zum Hybridisieren mit dem/den Polynukleotid(en) sind, das/die den/die Selektionsmarker kodiert/kodieren; bevorzugt sind die 20 oder mehr Nukleotide mindestens 90%, 95%, 97%, 98%, 99% oder sogar 100% komplementär zu und fähig zum Hybridisieren mit dem/den Polynukleotid(en), das/die den/die Selektionsmarker kodiert/kodieren.

10. Verfahren nach einem beliebigen der voranstehenden Ansprüche, wobei die Nuklease-Null-Variante eines Klasse-II-Cas9-Proteins eine Veränderung einer Aminosäure umfasst, die Position 10 und Position 840 von SEQ ID NO:2 entspricht; stärker bevorzugt umfasst die Variante eine Substitution von Asparaginsäure für Alanin, D10A, und eine Substitution von Histidin für Alanin, H840A.

11. Verfahren nach einem beliebigen der voranstehenden Ansprüche, wobei das mindestens eine Polynukleotid, das die gRNA kodiert, oder die mindestens zwei Polynukleotide, die die mindestens zwei gRNAs kodieren, im Genom der Wirtszelle teilweise oder vollständig durch das mindestens eine interessierende Polynukleotid oder die mindestens zwei verschiedenen interessierenden Polynukleotide ersetzt wurden; dadurch Inaktivieren des mindestens eine Polynukleotids, das die gRNA kodiert, oder der mindestens zwei Polynukleotide, die die mindestens zwei gRNAs kodieren.

## Revendications

1. Méthode pour insérer au moins un polynucléotide d'intérêt dans le génome d'une cellule hôte, la méthode comprenant les étapes de :

   a) fourniture d'une cellule hôte comprenant dans son génome :

   i. un polynucléotide codant un marqueur sélectionnable comprenant une séquence cible flanquée par une séquence PAM fonctionnelle pour une protéine Cas9 de classe II ;
   ii. au moins un polynucléotide codant un ARNg qui est complémentaire à au moins 80 % et capable de s'hybrider à la séquence cible ; et
   iii. un polynucléotide codant un variant à activité de nucléase nulle d'une protéine Cas9 de classe II capable d'interagir avec l'ARNg et se liant à la séquence cible, moyennant quoi l'expression du marqueur sélectionnable est réprimée ;

   b) transformation de ladite cellule hôte avec au moins un polynucléotide d'intérêt et capable d'inactiver l'au moins un polynucléotide codant l'ARNg ;
   c) sélection du trait conféré par le marqueur sélectionnable ; et
   d) identification d'une cellule hôte transformée,

   dans laquelle l'au moins un polynucléotide codant l'ARNg a été inactivé par l'au moins un polynucléotide d'intérêt.

2. Méthode pour insérer au moins deux polynucléotides d'intérêt différents dans le génome d'une cellule hôte, la méthode comprenant les étapes de :

   a) fourniture d'une cellule hôte comprenant dans son génome :

   i. au moins deux polynucléotides codant au moins deux marqueurs sélectionnables, comprenant chacun une séquence cible différente flanquée par une séquence PAM fonctionnelle pour une protéine Cas9 de classe II ;
   ii. au moins deux polynucléotides codant au moins deux ARNg qui sont complémentaires à au moins 80 % et capables de s'hybrider aux au moins deux séquences cibles différentes ;

iii. un polynucléotide codant un variant à activité de nucléase nulle d'une protéine Cas9 de classe II capable d'interagir avec les au moins deux ARNg et se liant aux au moins deux séquences cibles différentes, moyennant quoi l'expression des deux marqueurs sélectionnables différents est réprimée ;

b) transformation de ladite cellule hôte avec au moins deux polynucléotides d'intérêt différents, lesdits polynucléotides étant capables d'inactiver les au moins deux polynucléotides codant les au moins deux ARNg ; et
c) sélection des traits conférés par les au moins deux marqueurs sélectionnables différents ; et
d) identification d'une cellule hôte transformée,

dans laquelle les au moins deux polynucléotides codant les au moins deux ARNg ont été inactivés par les au moins deux polynucléotides d'intérêt différents.

3. Méthode selon l'une quelconque des revendications précédentes, dans laquelle l'au moins un polynucléotide ou les au moins deux polynucléotides d'intérêt code(nt) une protéine ; de préférence l'au moins un polynucléotide ou les au moins deux polynucléotides d'intérêt code(nt) une enzyme indépendamment choisie dans le groupe constitué d'une hydrolase, une isomérase, une ligase, une lyase, une oxydoréductase, et une transférase ; de manière plus particulièrement préférée une aminopeptidase, une amylase, une carbohydrase, une carboxypeptidase, une catalase, une cellobiohydrolase, une cellulase, une chitinase, une cutinase, une cyclodextrine glycosyltransférase, une désoxyribonucléase, une endoglucanase, une estérase, une alpha-galactosidase, une bêta-galactosidase, une glucoamylase, une alpha-glucosidase, une bêta-glucosidase, une invertase, une laccase, une lipase, une mannosidase, une mutanase, une oxydase, une enzyme pectinolytique, une peroxydase, une phosphodiestérase, une phytase, une polyphénol oxydase, une enzyme protéolytique, une ribonucléase, une transglutaminase, une xylanase, et une bêta-xylosidase.

4. Méthode selon l'une quelconque des revendications précédentes, dans laquelle la cellule hôte est une cellule hôte Gram positive ; de préférence la cellule hôte est choisie dans le groupe constitué des cellules hôtes de *Bacillus, Streptomyces, Streptococcus,* et *Lactobacillus* ; de manière particulièrement préférée la cellule hôte est choisie dans le groupe constitué par les cellules de *Bacillus alkalophilus, Bacillus altitudinis, Bacillus amyloliquefaciens, B. amyloliquefaciens* subsp. *plantarum, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus methylotrophicus, Bacillus pumilus, Bacillus safensis, Bacillus stearothermophilus, Bacillus subtilis,* et *Bacillus thuringiensis* ; de manière plus particulièrement préférée la cellule hôte procaryote est une cellule hôte de *Bacillus licheniformis.*

5. Méthode selon l'une quelconque des revendications 1 à 3, dans laquelle la cellule hôte est une cellule hôte fongique choisie dans le groupe constitué des cellules *d'Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* et *Trichoderma* ; de préférence la cellule hôte fongique est choisie dans le groupe constitué des cellules *d'Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* et *Trichoderma viride.*

6. Méthode selon l'une quelconque des revendications 1 à 3, dans laquelle la cellule hôte est une cellule hôte de levure choisie dans le groupe constitué des cellules de *Candida, Hansenula, Kluyveromyces, Pichia, Saccharomyces, Schizosaccharomyces,* et *Yarrowia* ; de préférence la cellule hôte est choisie dans le groupe constitué des cellules de *Kluyveromyces lactis, Pichia pastoris, Saccharomyces carlsbergensis, Saccharomyces cerevisiae, Saccharomyces diastaticus, Saccharomyces douglasii, Saccharomyces kluyveri, Saccharomyces norbensis, Saccharomyces*

*oviformis,* et *Yarrowia lipolytica.*

7. Méthode selon l'une quelconque des revendications précédentes, dans laquelle le marqueur sélectionnable ou les au moins deux marqueurs sélectionnables sont, indépendamment, un marqueur de sélection positive, un marqueur de sélection négative, un marqueur bidirectionnel, ou un gène conditionnellement essentiel.

8. Méthode selon l'une quelconque des revendications précédentes, dans laquelle le marqueur sélectionnable ou les au moins deux marqueurs sélectionnables sont indépendamment choisis dans le groupe de gènes constitué de *cat, erm, tet, amp, spec, kana, neo, dal, lysA, araA, galE, antK, metC, xylA, gntP, glpD, glpF, glpK, glpP, lacA2, hisC, gapA,* et *aspB.*

9. Méthode selon l'une quelconque des revendications précédentes, dans laquelle l'ARNg ou les au moins deux ARNg comprennent un premier ARN comprenant 20 nucléotides ou plus qui sont complémentaires à au moins 85 % et capables de s'hybrider au(x) polynucléotide(s) codant le ou les marqueurs sélectionnables ; de préférence les 20 nucléotides ou plus sont complémentaires à au moins 90 %, 95 %, 97 %, 98 %, 99 % ou même 100 % et capables de s'hybrider au(x) polynucléotide(s) codant le ou les marqueurs sélectionnables.

10. Méthode selon l'une quelconque des revendications précédentes, dans laquelle le variant à activité de nucléase nulle d'une protéine Cas9 de classe II comprend une altération d'un acide aminé correspondant à la position 10 et à la position 840 de la SEQ ID NO : 2 ; de préférence ledit variant comprend un remplacement d'acide aspartique par l'alanine, D10A, et un remplacement d'histidine par l'alanine, H840A.

11. Méthode selon l'une quelconque des revendications précédentes, dans laquelle l'au moins un polynucléotide codant l'ARNg ou les au moins deux polynucléotides codant les au moins deux ARNg ont été partiellement ou entièrement remplacés dans le génome de la cellule hôte par l'au moins un polynucléotide d'intérêt ou les au moins deux polynucléotides d'intérêt différents, inactivant ainsi l'au moins un polynucléotide codant l'ARNg ou les au moins deux polynucléotides codant les au moins deux ARNg.

Fig. 1

gnt-dsRED-gDNA(cat)
3133 bp

Fig. 2

**amyL-dsRED-gDNA(cat)**
2813 bp

Fig. 3

**lacA2-dsRED-gDNA(cat)**
3045 bp

Fig. 4

Fig. 5

**amyL-amyL**
3594 bp

Fig. 6

Fig. 7

Fig. 8

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9943835 A
- WO 9600787 A
- WO 0056900 A
- US 6011147 A
- WO 9425612 A
- WO 9533836 A
- WO 2010039889 A
- WO 0024883 A
- EP 238023 A
- EP 0506780 A
- EP 0805867 B1
- WO 199414968 A
- WO 2014052630 A
- EP 2029732 B1
- US 20130177942 A
- WO 2005042750 A
- US 5843720 A
- WO 200200907 A
- WO 2008066931 A
- WO 1993010249 A
- WO 2006042548 A
- WO 1999043835 A
- WO 2005098016 A
- WO 2007138049 A
- WO 2018077796 A

### Non-patent literature cited in the description

- **SU et al.** *Scientific Reports,* 2016, vol. 6, 37895
- **ALTENBUCHNER.** *Applied and Environmental Microbiology,* 2016, vol. 82, 5421-5427
- **PETERS et al.** *Current Opinion in Microbiology,* 2015, vol. 27, 121-126
- **ARAVIND ; KOONIN.** *Genome Research,* 2001, vol. 11, 1365-1374
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **INNIS et al.** PCR: A Guide to Methods and Application. Academic Press, 1990
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** Useful proteins from recombinant bacteria. *Scientific American,* 1980, vol. 242, 74-94
- **ROMANOS et al.** *Yeast,* 1992, vol. 8, 423-488
- **HUE et al.** *Journal of Bacteriology,* 1995, vol. 177, 3465-3471
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol. (Praha),* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207

- **BUCKLEY et al.** *Appl. Environ. Microbiol.,* 1999, vol. 65, 3800-3804
- **CLEWELL.** *Microbiol. Rev.,* 1981, vol. 45, 409-436
- **HAWKSWORTH et al.** Ainsworth and Bisby's Dictionary of The Fungi. University Press, 1995
- Biology and Activities of Yeast. Soc. App. Bacteriol. Symposium Series. 1980
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* vol. 78, 147-156
- Guide to Yeast Genetics and Molecular Biology. Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- **MAKAROVA et al.** An updated evolutionary classification of CRISPR-Cas systems. *Nature,* vol. 13, 722-736
- **ZETSCHE et al.** *Cell,* 2015, vol. 163, 759-771
- **JINEK et al.** A programmable dual-RNA-guided DNA endonuclease in adaptive bacterial immunity. *Science,* 2012, vol. 337, 816-821
- **SHAH et al.** Protospacer recognition motifs. *RNA Biol,* 2013, vol. 10 (5), 891-899
- **YASBIN et al.** *J. Bacteriol.,* 1975, vol. 121, 296-304